# EUROPÄISCHE PATENTSCHRIFT

(11) **EP 0 863 122 B1**
(45) Veröffentlichungstag und Bekanntmachung des Hinweises auf die Patenterteilung: **28.06.2000**
(21) Anmeldenummer: 98102661.0
(22) Anmeldetag: 17.02.1998
(51) Int. Cl.: C07C 43/13, C07C 41/01, C07C 41/06

(54) **Verfahren zur Herstellung von Polyhydroxyethern**
Process for the preparation of polyhydroxyethers
Procédé pour la préparation de polyhydroxyéthers

(30) Priorität: 04.03.1997 DE 19708695
(43) Veröffentlichungstag der Anmeldung: 09.09.1998
(73) Patentinhaber: Degussa-Hüls Aktiengesellschaft, 60287 Frankfurt am Main (DE)
(72) Erfinder: Morawietz, Marcus, Dr., 63457 Hanau (DE); Arntz, Dietrich, Dr., 61440 Oberursel (DE); Höpp, Mathias, Dr., 63599 Biebergemünd-Kassel (DE)

(56) Entgegenhaltungen:
- EP-A- 0 799 815
- DATABASE CHEMABS CHEMICAL ABSTRACTS SERVICE, COLUMBUS, OHIO, US STN, XP002070582 & CS 197 741 B (G. GUBA ET AL)
- DATABASE CHEMABS CHEMICAL ABSTRACTS SERVICE, COLUMBUS, OHIO, US STN, XP002070583 & SU 387 959 A (S.E. LUNEVA ET AL)
- DATABASE CHEMABS CHEMICAL ABSTRACTS SERVICE, COLUMBUS, OHIO, US STN, XP002070584 & G. GUBA ET AL: PETROCHEMIA, Bd. 19, Nr. 6, 1979, Seiten 198-206,

## Beschreibung

Die Erfindung betrifft ein Verfahren zur Herstellung von Polyhydroxyethern. Das Verfahren basiert auf der Umsetzung eines mehrwertigen Alkohols mit einem α,β-ungesättigten Aldehyd oder Keton und Formaldehyd in Gegenwart eines Alkali- oder Erdalkalihydroxids unter Bedingungen der Aldol-/cannizzaro-Reaktion.

Polyhydroxyether vom Typ Di-, Tri- und Polypentaerythritol der allgemeinen Formel (HOCH₂)₃C-O-[C(CH₂OH)₂CH₂-O]ₙH sowie entsprechende Polyhydroxyether auf der Basis von Trimethylolethan (TME) und Trimethylolpropan (TMP) gewinnen als Rohstoffe für spezielle Harze, Schmieröle, PVC-Stabilisatoren und PVC-Weichmacher zunehmend Bedeutung. Da Polyhydroxyether der vorgenannten Art bisher nur als Nebenprodukte bei der Herstellung von Pentaerythritol, TME beziehungsweise TMP aus Acetaldehyd beziehungsweise Propionaldehyd oder Butyraldehyd und Formaldehyd durch kombinierte Aldol-/Cannizzaro-Reaktion in geringer Menge anfallen, hat es nicht an Versuchen gefehlt (siehe z. B. US-Patente 2,325,589 und 2,490,567), solche Polyhydroxyether gezielt herzustellen.

Die Ausbeute an Dipentaerylthritol als Nebenprodukt bei der Herstellung von Pentaerythritol läßt sich gemäß JP-A 8 176 048 etwas erhöhen, indem anstelle eines Teils Acetaldehyd Acrolein eingesetzt wird. Bei diesem Verfahren werden Acetaldehyd und gegebenenfalls ein Teil des Acroleins in wäßrigem Medium vorgelegt und Formaldehyd sowie Acrolein zugetropft und die Umsetzung bei 0 bis 50 °C in Gegenwart eines Alkalihydroxids durchgeführt. Die Ausbeute an Dipenta, bezogen auf Acetaldehyd, wurde mit 21 bis 26 % angegeben. Gemäß dem in diesem Verfahren beschriebenen Vergleichsbeispiel - Umsetzung von Pentaerythritol mit Acrolein und Formaldehyd bei 70 °C - wurde Dipentaerythritol in einer Ausbeute von 8,5 % erhalten, und zudem entstanden Polymere von Acrolein.

Das CS-Patent 197 741 beschreibt ein Verfahren zur Herstellung von Dipentaerythritol durch Umsetzung von Pentaerythritol mit Arcolein und Formaldehyd bei 0 bis 50 °C, einem Molverhältnis von Pentaerythritol zu Acrolein von 0,2 bis 3 und einem Molverhältnis von Formaldehyd zu Acrolein von 1 bis 10. Die gesamte Menge Formaldehyd wird vorgelegt, Acrolein wird zugegeben; Pentaerythritol wird zu Beginn oder während der Umsetzung zugegeben. Bei der Nacharbeitung des Verfahrens wurde anstelle der im Patent angegebenen hohen Ausbeute an Dipentaerythritol nur eine geringe Ausbeute erzielt - siehe Vergleichsbeispiel 1.

Ein ähnliches Verfahren wie dasjenige des CS-Patents lehrt das SU-Patent 387 959: Hier werden 0,1 bis 2 Mol Pentaerythritol pro Mol Acrolein eingesetzt und bei 50 bis 90 °C mit Formaldehyd umgesetzt; das Molverhältnis Acrolein zu Formaldehyd zu Alkalihydroxid beträgt 1 zu 3 bis 7 zu 0,25 bis 2. Vorgelegt wird Pentaerythritol in Wasser; zugetropft werden Acrolein und Formaldehyd. Auch bei der Nacharbeitung dieses Verfahrens waren die gefundene Ausbeute an Di- und Tripentaerythritol und die Gesamtausbeute wesentlich niedriger als im Patent angegeben - siehe Vergleichsbeispiel 2.

Aufgabe der vorliegenden Erfindung ist demgemäß, ein Verfahren aufzuzeigen, das es gestattet, unter Anwendung des am Beispiels Dipentaerythritol zuvor dargelegten Verfahrensprinzips Dipentaerythritol in höherer Selektivität zu gewinnen. Zudem sollten die Nebenprodukte Tri- und höhere Polypentaerythritole in geringerem Umfang gebildet werden als im Verfahren des vorgenannten SU-Dokuments.

Gefunden wurde ein Verfahren zur Herstellung von Polyhydroxyethern der allgemeinen Formel (I)

(HO)_{(w-m)}W(OV)ₘ (I),

worin W für einen aliphatische und/oder cycloaliphatische Strukturmerkmale aufweisenden organischen Rest eines primäre und/oder sekundäre Hydroxylgruppen enthaltenden organischen mehrwertigen Alkohols der allgemeinen Formel W(OH)_{w} (II), W für eine ganze Zahl von 2 bis 1000 und m für eine ganze Zahl zwischen 1 bis w stehen und OV ausgewählt ist aus hydroxylgruppenhaltigen Strukturelementen der allgemeinen Formel (III) 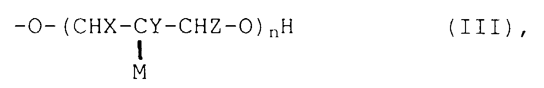 worin bedeuten
- X:: H, C₁- bis C₆-Alkyl, Aryl, Alkoxymethyl, Aryloxymethyl;
- Y:: H, C₁- bis C₆-Alkyl, Aryl, Alkoxymethyl, Aryloxymethyl, Carbalkoxymethyl, Carbamidomethyl oder das Strukturmerkmal M;
- Z:: H, C₁- bis C₆-Alkyl, Aryl oder X-Z zusammen eine C₂- oder C₃-Alkylengruppe;
- M:: -CH₂OH oder 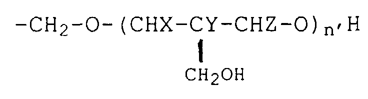
- n,n':: eine ganze Zahl von 1 bis 5, und
wobei Alkyl und Aryl in X, Y und Z auch substituiert und die m Strukturelemente OV im Polyhydroxyether (I) gleich oder verschieden sein können,
umfassend Umsetzen eines mehrwertigen Alkohols der allgemeinen Formel W(OH)_{w} (II) mit einer α,β-ungesättigten Carbonylverbindung der allgemeinen Formel

XCH=CY-CZ=O (IV),

worin X, Y, und Z mit der Ausnahme, daß Y nicht für das Strukturmerkmal M steht, die vorgenannte Bedeutung haben, und Formaldehyd in Gegenwart eines Alkali- oder Erdalkalihydroxids in wäßriger Phase bei einem pH-Wert oberhalb 7 und einer Temperatur im Bereich von 0 bis 100 °C, wobei das Molverhältnis von II zu IV 0,05 bis 20, das Molverhältnis von Formaldehyd zu IV 2 bis 15 und das Molverhältnis von Metallhydroxidäquivalent zu IV 1 bis 3 beträgt, Ansäuern des Reaktionsgemischs, und Isolieren der Reaktionsprodukte, das dadurch gekennzeichnet ist, daß man in eine wäßrige Lösung, enthaltend mindestens 10 Mol-% des mehrwertigen Alkohols (II), mindestens 10 bis 70 Mol-% des Formaldehyds und mindestens 10 Mol-% des Alkali- oder Erdalkalihydroxids, die α,β-ungesättige Carbonylverbindung (IV) und den restlichen Formaldehyd, den restlichen mehrwertigen Alkohol (II) und restliches Alkali- oder Erdalkalihydroxid kontinuierlich oder periodisch derart einträgt, daß im Reaktionsgemisch Formaldehyd stets im Überschuß gegenüber dem α,β-ungesättigten Aldehyd oder Keton (IV) vorliegt.

Die Unteransprüche richten sich auf bevorzugte Ausführungsformen des Verfahrens. Erfindungswesentliches Merkmal ist, daß das Reaktionssystem vor Zugabe des α,β-ungesättigten Aldehyds oder Ketons bereits einen Teil des für die Umsetzung erforderlichen Formaldehyds enthält. Durch diese Maßnahme wird eine Polymerisation der α,β-ungesättigten Carbonylverbindung vermieden und zudem ist der Polyhydroxyether in höherer Ausbeute erhältlich als dies bei den vorbekannten Verfahren möglich war.

Der zu verwendende mehrwertige Alkohol der Formel W(HO)_{w} (II) kann monomer, oligomer oder polymer sein und primäre oder/und sekundäre Hydroxylgruppen, wobei w eine ganze Zahl von 2 bis 1000 ist, enthalten. Der organische Rest W, der aliphatische und/oder cycloaliphatische Strukturmerkmale aufweisen kann, kann außer mindestens zwei Hydroxylgruppen auch andere funktionelle Gruppen aufweisen, wie beispielsweise Gruppen aus der Reihe -O-, -COOMe (Salze), -COOR, -SO₃Me (Salze), -SO₃R, -PO₃Me₂, -NR₂, Hal, CN, -S(O)-, -SO₂-, wobei R für Alkyl und Me für ein Alkalimetall- oder Ammoniumion steht.

Unter aliphatischen beziehungsweise cycloaliphatischen monomeren Alkoholen (II) sind beispielhaft 2-, 3-, 4-, 5- und 6-wertige Alkohole zu nennen, z. B. solche aus der Reihe Ethylenglykol (EG), 1,2- und 1,3-Propylenglykol (1,2-PD und 1,3-PD), Butan-1,4-diol (1,4-BD), Hexan-1,6-diol (1,6-HD), Neopentylglykol (NPG), 2-Methyl-1,3-propandiol (2-Methyl-1,3-PD), 2,2,4-Trimethyl-1,3-pentandiol, 1,4- und 1,2-Cyclohexandiol, Weinsäure, Diethanolamin, Oxaheptandiol, Cyclohexan-1,4-dimethanol, Dimethylolessigsäure; Glycerin, Trimethylolethan (TME), Trimethylolpropan (TMP), Trimethylolessigsäure; Diglycerin, Pentaerythritol (Penta), Di-TME, Di-TMP (= 2,6-Bis(hydroxymethyl)-2,6-diethyl-4-oxa-1,6-heptandiol), Dipentaerythritol (Dipenta = 2,2,6,6-Tetrakis(hydroxymethyl)-4-oxa-1,6-heptandiol), Triglycerin, Tripentaerythritol (Tripenta), Tri-TMP, Tri-TME; Mono- und Disaccharide. Besonders bevorzugte monomere Alkohole II sind solche mit 2 bis 6 primären Hydroxylgruppen. Unter den polymeren Verbindungen der Formel II sind beispielhaft Polyvinylalkohol, Stärke, Polysaccharide und Poly(meth)acrylate und Copolymere davon, enthaltend als Monomereinheit ein Hydroxyalkyl(meth)acrylat, zu nennen.

Bei der erfindungsgemäß einzusetzenden α,β-ungesättigten Carbonylverbindung (IV) kann es sich um Aldehyde oder Ketone handeln. Aldehyde, also Verbindungen (IV) mit Z gleich H, werden bevorzugt. Die Reste X und Y können in IV auch unter den Reaktionsbedingungen stabile Substituenten aufweisen, wie beispielsweise -N Alkyl₂, -N Aryl₂, -OC(O)Alkyl, -OC(O)Aryl, -COO Alkyl, -COO Aryl, -CON Alkyl₂. Während X und Y in der Verbindung IV die zuvorgenannte Bedeutung, insbesondere H, C₁- bis C₄-Alkyl und Aryl, haben können, werden Verbindungen mit X und vorzugsweise auch Y gleich H bevorzugt. Beispiele für α,β-ungesättigte Ketone sind Vinyl-alkylketon, wie Vinylmethylketon, und Vinyl-arylketon, wie Vinyl-phenylketon. Beispiele für α,β-ungesättigte Aldehyde (IV) sind: Acrolein, Methacrolein, 2-Ethylacrolein, 2-n-Propyl-, 2-n-Butyl- und 2-Isobutylacrolein; 2-Arylacrolein, wie z. B. 2-Phenylacrolein, 2-p-Tolylacrolein, 2-p-Hydroxyphenylacrolein; 2-Alkoxymethylacrolein, wie 2-Methoxylmethylacrolein, 2-Aryloxymethylacrolein, wie 2-Phenoxymethylacrolein.

Besonders bevorzugt werden ein 2- bis 6-wertiger Alkohol (II) mit primären Hydroxylgruppen und ein α,β-ungesättigter Aldehyd (IV) mit X gleich H und Y gleich H oder C₁- bis c₄-Alkyl oder Phenyl umgesetzt.

Das Reaktionsprinzip - siehe Formelschema - gestattet es, symmetrische und unsymmetrische Polyhydroxyether herzustellen. Während symmetrische Produkte bekannt sind, handelt es sich bei den unsymmetrischen Produkten der Formel I um neue Verbindungen.

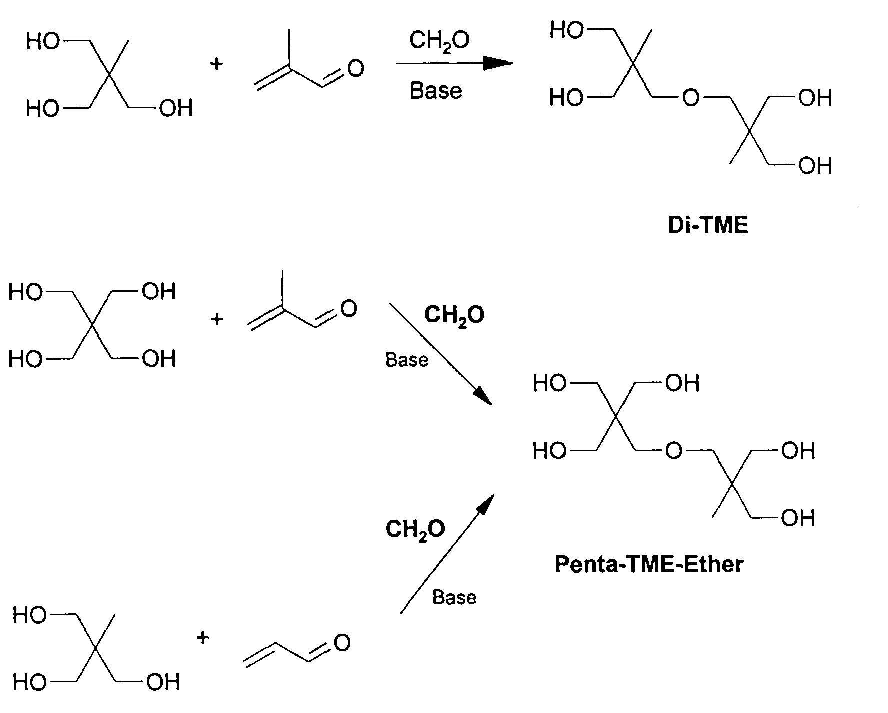

Während im vorbekannten Verfahren gemäß SU-Patent 387 959 aus Pentaerythritol und Acrolein als Nebenprodukte hauptsächlich Tri- und Polypentaerythritole gebildet werden, wird im erfindungsgemäßen Verfahren eine viel höhere Dipentaerythritol-Selektivität erzielt; gebildetes Pentaerythritol kann rezykliert werden.

Formaldehyd für die Aldolkondensation als auch Reduktionsmittel in der Cannizzaro-Reaktion kann in jeder verfügbaren Form verwendet werden, vorzugsweise als wäßrige Lösung mit einem Gehalt von 15 bis 60 Gew.-%, insbesondere 15 bis 30 Gew.-%. Während mindestens 10 Mol-% des erforderlichen Formaldehyds vorgelegt werden müssen, werden zweckmäßigerweise bis zu 50 Mol-%, besonders bevorzugt 20 bis 50 Mol-%, ganz besonders 20 bis 30 Mol-%, vorgelegt und die restliche Menge zusammen mit der α,β-ungesättigten Carbonylverbindung eingetragen.

Dieses Eintragen kann kontinuierlich oder periodisch erfolgen. Das Molverhältnis Formaldehyd zu α,β-ungesättigter Carbonylverbindung (IV) liegt im allgemeinen bei mindestens 2 zu 1 bei Y ungleich H und mindestens 3 zu 1 bei Y gleich H. Formaldehyd kann auch in hohem Überschuß, etwa bis zu 15 zu 1 eingesetzt werden. Nach beendeter Umsetzung noch anwesender Formaldehyd kann entweder mittels Wasserdampfdestillation oder durch Druckdestillation vom Reaktionsgemisch abgetrennt werden.

Der mehrwertige Alkohol kann in Form einer wäßrigen Lösung oder einer wäßrigen Suspension vorgelegt werden. Zweckmäßigerweise wird der Alkohol in einer Menge von 50 bis 100 Mol-% vorgelegt und der Rest portionsweise oder kontinuierlich während der Zugabe der α,β-ungesättigten Carbonylverbindung zugegeben; und zwar als Lösung oder Suspension oder als Feststoff oder Flüssigkeit.

Das Molverhältnis mehrwertiger Alkohol zu α,β-ungesättigter Carbonylverbindung richtet sich nach der herzustellenden Struktur des Polyhydroxyethers I sowie dem Oligomerisierungsgrad n und n' im Strukturelement III sowie der Zahl für m. Bei mehrwertigen Alkoholen II mit 2 bis 6 Hydroxylgruppen wird die α,β-ungesättigte Carbonylverbindung IV üblicherweise im Molverhältnis II zu IV im Bereich von 5 bis 0,2 umgesetzt.

Als Katalysator für die Cannizzaro-Reaktion wird eine Base, im allgemeinen ein Alkali- oder Erdalkalihydroxid eingesetzt, wie NaOH, KOH und Ca(OH)₂. Das Molverhältnis Metallhydroxid-Äquivalent zu α,β-ungesättigter Carbonylverbindung liegt vorzugsweise zwischen 1 zu 1 und 3 zu 1. Der Katalysator kann insgesamt vorgelegt werden, oder ein Teil wird während der Umsetzung zudosiert.

Die Vorlage enthält Wasser; das Molverhältnis Wasser zu α,β-ungesättigter Carbonylverbindung liegt bevorzugt zwischen 10 zu 1 bis 300 zu 1, insbesondere zwischen 20 zu 1 bis 150 zu 1.

Die Zugabe der α,β-ungesättigten Carbonylverbindung, des restlichen Formaldehyds und des Katalysators zu der Vorlage kann simultan oder hintereinander erfolgen. Die Rohstoffe können vorgemischt oder separat zugegeben werden. Einzuhalten ist aber ein ausreichender Überschuß an Formaldehyd in der Vorlage.

Die Zugabezeit des Formaldehyds, der Polyhydroxyverbindung und der α,β-ungesättigten Carbonylverbindung liegt meistens zwischen 0,1 und 5 h, typischerweise zwischen 0,5 bis 3 h. Nach der erfolgten Rohstoffzugabe schließt sich meist eine Nachreaktion von bis zu 4 h an.

Das erfindungsgemäße Verfahren wird bei Temperaturen im Bereich 0 bis 100 °C durchgeführt und bevorzugt im Bereich 20 bis 60 °C. Die Reaktion kann bei konstanter, steigender oder fallender Temperatur durchgeführt werden. In der Phase nach der Rohstoffzugabe wird das Gemisch bei 20 bis 70 °C, vorzugsweise bei 30 bis 60 °C gerührt.

Der pH wird während der Umsetzung bei 7 bis 14, insbesondere 10 bis 14 gehalten. Für eine optimale Ausbeute an Polyhydroxyethern hat es sich als vorteilhaft erwiesen, den Katalysator, die Polyhydroxyverbindung und 10 bis 30 % des Formaldehyds vollständig in Wasser zu lösen und die restlichen 70 bis 90 % des Formaldehys und die α,β-ungesättigte Carbonylverbindung parallel oder vorgemischt kontinuierlich zuzugeben. Die Reaktions- beziehungsweise Zugabezeit beträgt 1 bis 3 h und die Reaktionstemperatur ist 30 bis 60 °C.

An die Umsetzung schließt sich eine Neutralisation an, um die Base in ein Salz zu überführen. Zur Neutralisation eignet sich insbesondere Ameisensäure.

Zur Aufarbeitung wird der noch vorhandene Formaldehyd destillativ, etwa mittels einer Druckdestillation oder Wasserdampfdestillation, aus dem Reaktionsgemisch entfernt und der Polyhydroxyether durch Kristallisation von den entstandenen Metallformiaten durch Kristallisation abgetrennt und anschließend fraktioniert kristallisiert.

Erfindungsgemäß lassen sich unterschiedlich strukturierte Polyhydroxyether herstellen. Beispiele erfindungsgemäßer unsymmetrischer Polyhydroxyether mit unterschiedlichem Kohlenstoffgerüst für W und die monomere Einheit von OV unter Einsatz von Acrolein (a), Methacrolein (b) und Ethylacrolein (c) sind der folgenden Liste zu entnehmen. Derartige unsymmetrische Polyhydroxyether erweitern die Möglichkeiten, um hieraus Folgeprodukte aus der Reihe der Harze, Schmieröle, PVC-Stabilisatoren und Weichmacher mit spezifischen Eigenschaften zu erzeugen. Ein Vorteil der Schmieröle auf Basis der unsymmetrischen Polyhydroxyether ist ihr tieferer Stockpunkt. Zudem ist die Löslichkeit der unsymmetrischen Polyhydroxyether höher als jene der symmetrischen. Erfindungsgemäße Polyhydroxyether auf der Basis eines hydroxylgruppenhaltigen Polymeren, wie Umsetzungsprodukten von Stärke mit Acrolein und Formaldehyd eignen sich als Cobuilder und Superabsorbentien.

### a) Polyhydroxyether (I) auf Basis Acrolein:

Penta-TME-Ether, Penta-TMP-Ether, Penta-Di-TME-Ether, Penta-Di-TMP-Ether, Penta-Glycerin-Ether, Penta-NPG-Ether Penta-1,2-PD-Ether, Penta-1,3-PD-Ether, Penta-EG-Ether Penta-2-Methyl-1,3-PD-Ether, Penta-1,4-BD-Ether Penta-1,6-HD-Ether, Penta-1,4-Cyclohexandimethanol-Ether Penta-2,2,4-Trimethyl-1,3-pentandiol-Ether, Penta-Saccharid-Ether, Penta-Polysaccharid-Ether
Bis(Penta)-Di-TME-Ether, Bis(Penta)-Di-TMP-Ether Bis(Penta)-TME-Ether, Bis(Penta)-TMP-Ether, Bis(Penta)-Glycerin-Ether, Bis(Penta)-NPG-Ether, Bis(Penta)-1,2-PD-Ether, Bis(Penta)-1,3-PD-Ether, Bis(Penta)-EG-Ether Bis(Penta)-2-Methyl-1,3-PD-Ether, Bis(Penta)-1,4-BD-Ether Bis(Penta)-1,6-HD-Ether, Bis(Penta)-1,4-Cyclohexandimethanol-Ether, Bis(Penta)-2,2,4-Trimethyl-1,3-pentandiol-Ether, Bis(Penta)-Saccharid-Ether, Bis(Penta)-Polysaccharid-Ether
Tris(Penta)-Di-TME-Ether, Tris(Penta)-Di-TMP-Ether Tris(Penta)-TME-Ether, Tris (Penta)-TMP-Ether, Tris(Penta)-Glycerin-Ether, Tris(Penta)-Saccharid-Ether, Tris(Penta)-Polysaccharid-Ether, Tetrakis(Penta)-Di-TME-Ether Tetrakis(Penta)-Di-TMP-Ether, Tetrakis(Penta)-Saccharid-Ether, Tetrakis(Penta)-Polysaccharid-Ether
Poly(Penta)-Polysaccharid-Ether

### b) Polyhydroxyether (I) auf Basis Methacrolein:

TME-Tripenta-Ether, TME-Dipenta-Ether, TME-Di-TMP-Ether, TME-TMP-Ether, TME-Glycerin-Ether, TME-NPG-Ether TME-1,2-PD-Ether, TME-1,3-PD-Ether, TME-EG-Ether, TME-2-Methyl-1,3-PD-Ether, TME-1,4-BD-Ether, TME-1,6-HD-Ether TME-1,4-Cyclohexandimethanol-Ether, TME-2,2,4-Trimethyl-1,3-pentandiol-Ether, TME-Saccharid-Ether, TME-Polysaccharid-Ether
Bis(TME)-Tripenta-Ether, Bis(TME)-Dipenta-Ether, Bis(TME)-Di-TMP-Ether, Bis(TME)-Penta-Ether, Bis(TME)-TMP-Ether Bis(TME)-Glycerin-Ether, Bis(TME)-NPG-Ether, Bis(TME)-1,2-PD-Ether, Bis(TME)-1,3-PD-Ether, Bis(TME)-EG-Ether, Bis(TME)-2-Methyl-1,3-PD-Ether, Bis(TME)-1,4-BD-Ether Bis(TME)-1,6-HD-Ether, Bis(TME)-1,4-Cyclohexandimethanol-Ether, Bis(TME)-2,2,4-Trimethyl-1,3-pentandiol-Ether Bis(TME)-Saccharid-Ether, Bis(TME)-Polysaccharid-Ether
Tris(TME)-Tripenta-Ether, Tris(TME)-Dipenta-Ether, Tris(TME)-Di-TMP-Ether, Tris(TME)-Penta-Ether, Tris(TME)-TMP-Ether, Tris(TME)-Glycerin-Ether, Tris(TME)-Saccharid-Ether, Tris(TME)-Polysaccharid-Ether
Tetrakis(TME)-Tripenta-Ether, Tetrakis(TME)-Dipenta-Ether Tetrakis(TME)-Di-TMP-Ether, Tetrakis(TME)-Penta-Ether Tetrakis(TME)-Saccharid-Ether, Tetrakis(TME)-Polysaccharid-Ether
Pentakis(TME)-Tripenta-Ether, Pentakis(TME)-Dipenta-Ether, Pentakis(TME)-Polysaccharid-Ether
Hexakis(TME)-Tripenta-Ether, Hexakis(TME)-Dipenta-Ether, Hexakis(TME)-Polysaccharid-Ether
Heptakis(TME)-Tripenta-Ether, Heptakis(TME)-Polysaccharid-Ether
Octakis(TME)-Tripenta-Ether, Octakis(TME)-Polysaccharid-Ether
Poly(TME)-Polysaccharid-Ether

### c) Polyhydroxyether (I) auf Basis Ethylacrolein:

TMP-Tripenta-Ether, TMP-Dipenta-Ether, TMP-Di-TME-Ether, TMP-Glycerin-Ether, TMP-NPG-Ether, TMP-1,2-PD-Ether TMP-1,3-PD-Ether, TMP-EG-Ether, TMP-2-Methyl-1,3-PD-Ether TMP-1,4-BD-Ether, TMP-1,6-HD-Ether, TMP-1,4-Cyclohexandimethanol-Ether, TMP-2,2,4-Trimethyl-1,3-pentandiol-Ether, TMP-Saccharid-Ether, TMP-Polysaccharid-Ether
Bis(TMP)-Tripenta-Ether, Bis(TMP)-Dipenta-Ether Bis(TMP)-Di-TME-Ether, Bis(TMP)-Penta-Ether, Bis(TMP)-TME-Ether, Bis(TMP)-Glycerin-Ether, Bis(TMP)-NPG-Ether, Bis(TMP)-1,2-PD-Ether, Bis(TMP)-1,3-PD-Ether, Bis(TMP)-EG-Ether, Bis(TMP)-2-Methyl-1,3-PD-Ether, Bis(TMP)-1,4-BD-Ether, Bis(TMP)-1,6-HD-Ether, Bis(TMP)-1,4-Cyclohexandimethanol-Ether, Bis(TMP)-2,2,4-Trimethyl-1,3-pentandiol-Ether, Bis(TMP)-Saccharid-Ether, Bis(TMP)-Polysaccharid-Ether
Tris(TMP)-Tripenta-Ether, Tris(TMP)-Dipenta-Ether Tris(TMP)-Di-TME-Ether, Tris(TMP)-Penta-Ether, Tris(TMP)-TME-Ether, Tris(TMP)-Glycerin-Ether, Tris(TMP)-Saccharid-Ether, Tris(TMP)-Polysaccharid-Ether
Tetrakis(TMP)-Tripenta-Ether, Tetrakis(TMP)-Dipenta-Ether Tetrakis(TMP)-Di-TME-Ether, Tetrakis(TMP)-Penta-Ether Tetrakis(TMP)-Saccharid-Ether, Tetrakis(TMP)-Polysaccharid-Ether
Pentakis(TMP)-Tripenta-Ether, Pentakis(TMP)-Dipenta-Ether Pentakis(TMP)-Polysaccharid-Ether
Hexakis(TMP)-Tripenta-Ether, Hexakis(TMP)-Dipenta-Ether Hexakis(TMP)-Polysaccharid-Ether
Heptakis(TMP)-Tripenta-Ether, Heptakis(TMP)-Polysaccharid-Ether
Octakis(TMP)-Tripenta-Ether, Octakis(TMP)-Polysaccharid-Ether
Poly(TMP)-Polysaccharid-Ether

Anhand der nachfolgenden Vergleichsbeispiele und erfindungsgemäßen Beispiele wird die Erfindung weiter verdeutlicht.

### Vergleichsbeispiel 1

### Herstellung von Dipentaerythritol gemäß Beispiel 1 des CS-Patents 197741

In einem Kolben wurden 870 g Wasser, 48 g (1,2 mol) NaOH, 140 g (1,03 mol) Pentaerythritol und 400 g (4,0 mol) wäßrige 30 % Formaldehyd-Lösung bei 40 °C vorgelegt. Bei dieser Temperatur wurden 196,9 g einer wäßrigen 30 % Acrolein-Lösung innerhalb von 15 min. zugetropft. Nach der Acrolein-Zugabe wurde das Reaktionsgemisch 30 min. bei 40 °C gerührt und anschließend mit Ameisensäure auf pH = 6 angesäuert. Der noch vorhandene Formaldehyd wurde durch Wasserdampfdestillation entfernt. Die Formaldehyd-freie Lösung wurde vollständig eingedampft und der trockene Rückstand mittels Gaschromatographie analysiert.

| | |
|---|---|
| Eindampfrückstand | 365,1 g |
| Natriumformiat | 81,6 g |
| Organische Verbindungen | 283,5 g |

Die im folgenden erläuterte Bilanz zur Analytik und Ausbeute bezieht sich auf die eingesetzte Carbonylkomponente, hier also Acrolein, und bildet die Grundlage für alle weiteren Versuchsauswertungen:

| Polyol | Zusammensetzung des Rückstandes | | Ausbeute bezogen auf Acrolein (%) | Ausbeute laut CS 197741 (%) |
|---|---|---|---|---|
| | (%) | (g) | | |
| Pefo | 2,02 | 7,4 | 0,5 | k. A. |
| Penta | 56,07 | 204,7 | - | - |
| Penta (neu)* | - | 64,7 | 47,5 | 2,9 |
| Dipenta | 5,04 | 18,4 | 14,5 | 61,9 |
| Pe₂fo | 3,94 | 14,4 | 10,1 | k. A. |
| Tripenta | 0,85 | 3,1 | 2,5 | 17,1 |
| Tetrapenta | 0,33 | 1,2 | 1,0 | k. A. |
| Gesamtausbeute | 68,25 | 249,2 | 76,1 | 81,9 |

| | | | | |
|---|---|---|---|---|
| * Penta (neu)=(Penta im Reaktionsprodukt)-(Penta in der Vorlage) k. A. = keine Angabe Pefo = Pentaerythritolmonoformal, 5,5-Bis(hydroxymethyl)-1,3-dioxan Pe₂fo = Bispentaerythritolformal, 2,2,8,8-Tetrakis (hydroxymethyl)-4,6-dioxanonan-1,9-diol | | | | |

### Vergleichsbeispiel 2

### Herstellung von Dipentaerythritol gemäß SU-Patent 387 959

In einem 6 l-Reaktionsgefäß wurden 272 g (2 mol) Pentraerythritol und 350 g (19,4 mol) Wasser auf 70 °C temperiert. Zu dieser Lösung wurden innerhalb von 15 min. bei einer konstanten Temperatur von 70 °C 116 g (2 mol) Acrolein (96 %) und 2250 g (9 mol) 12 %ige Formaldehyd-Lösung und 168 g (3 mol) Kaliumhydroxid kontinuierlich und parallel zugetropft. Während der Reaktion färbte sich das Reaktionsgemisch rötlich. Nach den Zugaben wurde das Reaktionsgemisch noch 10 min. bei 70 °C gerührt. Danach wurde das Reaktionsgemisch mit Ameisensäure auf pH = 6 eingestellt. Der Formaldehyd wurde durch Wasserdampfdestillation entfernt und die Zusammensetzung der Reaktionslösung mittels Gaschromatographie analysiert. Es wurde ein Eindampfrückstand von 730 g erhalten. Die Zusammensetzung und Ausbeuten folgen aus der Tabelle:

| Polyol | Zusammensetzung des Rückstandes | | Ausbeute bezogen auf Acrolein (%) | Ausbeute laut SU 387 959 (%) |
|---|---|---|---|---|
| | (%) | (g) | | |
| Pefo | 0,22 | 1,6 | 0,5 | k. A. |
| Penta | 37,56 | 274,2 | - | - |
| Penta (neu)* | - | 2,2 | 0,8 | 2,9 |
| Dipenta | 13,06 | 95,3 | 37,5 | 41,8 |
| Pe₂fo | 0,58 | 4,3 | 1,5 | k. A. |
| Tripenta | 7,53 | 54,9 | 22,1 | 51,0 |
| Tetrapenta | 1,61 | 11,8 | 4,8 | k. A. |
| Gesamtausbeute | 60,56 | 442,1 | 67,2 | 95,7 |

| | | | | |
|---|---|---|---|---|
| * siehe Fußnote Vergleichsbeispiel 1 | | | | |

### Beispiel 1

### Herstellung von Dipentaerythritol

In einem 6 l-Reaktionsgefäß wurden 545,6 g (4 mol) wäßrige 22 % Formaldehyd-Lösung, 272 g (2 mol) Pentaerythritol, 3200 g (177 mol) Wasser, 96 g (2,4 mol) Natriumhydroxid auf 20 °C temperiert. Zu dieser Lösung wurden innerhalb von 15 min. bei einer konstanten Temperatur von 20 °C 116 g (2 mol) Acrolein (96 %) und 545,6 g (4 mol) 22 % Formaldehyd-Lösung kontinuierlich und parallel zugetropft. Anschließend wurden 272,2 g (2 mol) 22 % Formaldehyd-Lösung bei 20 °C in 30 min. auf 50 °C erwärmt und 30 min. bei dieser Temperatur gerührt. Danach wurde mit Ameisensäure die Lösung auf pH = 6 eingestellt und die Zusammensetzung der Reaktionslösung mittels Gaschromatographie analysiert. Es wurde ein Eindampfrückstand von 694 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 2,7 | 0,9 |
| Penta | 363,5 | - |
| Penta (neu) * | 91,5 | 33,6 |
| Dipenta | 102,0 | 40,1 |
| Pe₂fo | 13,36 | 4,7 |
| Tripenta | 24,3 | 9,8 |
| Tetrapenta | 3,7 | 1,5 |
| Gesamtausbeute | 509,6 | 90,6 |

| | | |
|---|---|---|
| * Penta (neu)=(Penta im Reaktionsprodukt)-(Penta in der Vorlage) | | |

### Beispiel 2

### Herstellung von Dipentaerythritol

In einem 6 l-Reaktionsgefäß wurden 272,8 g (2 mol) wäßrige 22 % Formaldehyd-Lösung, 272 g (2 mol) Pentaerythritol, 3200 g (177 mol) Wasser, 96 g (2,4 mol) Natriumhydroxid auf 40 °C temperiert. Zu dieser Lösung wurden innerhalb von 2 h bei einer konstanten Temperatur von 40 °C 116 g (2 mol) Acrolein (96 %) und 1091,2 g (8 mol) 22 % Formaldehyd-Lösung, die vorher über einen Mischer laufen, kontinuierlich zudosiert. Nach der Zugabe wurde das Reaktionsgemisch noch 30 min. bei der Temperatur gerührt und anschließend mit Ameisensäure auf pH = 6 eingestellt. Der Formaldehyd wurde durch Wasserdampfdestillation entfernt und die Zusammensetzung der Reaktionslösung mittels Gaschromatographie analysiert. Es wurde ein Eindampfrückstand von 701 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 1,5 | 0,5 |
| Penta | 348,8 | - |
| Penta (neu) * | 76,8 | 28,2 |
| Dipenta | 119,8 | 47,1 |
| Pe₂fo | 2,8 | 1,0 |
| Tripenta | 37,2 | 15,0 |
| Tetrapenta | 8,6 | 3,5 |
| Gesamtausbeute | 518,7 | 95,3 |

| | | |
|---|---|---|
| * siehe Fußnote Beispiel 1 | | |

### Beispiel 3

### Herstellung von Dipentaerythritol

Die Reaktion wurde analog dem Beispiel 2 durchgeführt, mit dem Unterschied, daß die Reaktionstemperatur 50 °C betrug und die Zugabe von Acrolein und Formaldehyd-Lösung innerhalb 1 h erfolgte. Es wurde ein Eindampfrückstand von 699 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 1,5 | 0,5 |
| Penta | 350,4 | - |
| Penta (neu) * | 78,4 | 28,8 |
| Dipenta | 124,8 | 49,1 |
| Pe₂fo | 2,3 | 0,8 |
| Tripenta | 31,0 | 12,5 |
| Tetrapenta | 8,1 | 3,3 |
| Gesamtausbeute | 518,1 | 95,0 |

| | | |
|---|---|---|
| * siehe Fußnote Beispiel 1 | | |

### Beispiel 4

### Herstellung von Dipentaerythritol

Die Reaktion wurde analog dem Beispiel 2 durchgeführt, mit dem Unterschied, daß anstelle des Natriumhydroxids 93,6 g (1,2 mol) Kalk eingesetzt wurden. Es wurde ein Eindampfrückstand von 715 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 2,1 | 0,7 |
| Penta | 346,9 | - |
| Penta (neu) * | 74,9 | 27,5 |
| Dipenta | 121,8 | 47,9 |
| Pe₂fo | 8,8 | 3,1 |
| Tripenta | 35,0 | 14,1 |
| Tetrapenta | 6,4 | 2,6 |
| Gesamtausbeute | 521,0 | 95,9 |

| | | |
|---|---|---|
| * siehe Fußnote Beispiel 1 | | |

### Beispiel 5

### Herstellung von Dipentaerythritol

Die Reaktion wurde analog dem Beispiel 3 durchgeführt, mit dem Unterschied, daß 544 g (4 mol) Pentaerythritol eingesetzt wurden. Es wurde ein Eindampfrückstand von 965 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 1,2 | 0,4 |
| Penta | 576,9 | - |
| Penta (neu) * | 32,9 | 12,1 |
| Dipenta | 149,8 | 58,9 |
| Pe₂fo | 11,37 | 4,0 |
| Tripenta | 29,5 | 11,9 |
| Tetrapenta | 6,3 | 2,6 |
| Gesamtausbeute | 775,1 | 89,9 |

| | | |
|---|---|---|
| * siehe Fußnote Beispiel 1 | | |

### Beispiel 6

### Herstellung von Dipentaerythritol

Die Reaktion wurde analog dem Beispiel 3 durchgeführt, mit dem Unterschied, daß 680 g (5 mol) Pentaerythritol eingesetzt wurden. Es wurde ein Eindampfrückstand von 1100 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 0,6 | 0,2 |
| Penta | 692,8 | - |
| Penta (neu) * | 12,8 | 4,7 |
| Dipenta | 170,6 | 67,1 |
| Pe₂fo | 6,8 | 2,4 |
| Tripenta | 41,9 | 16,9 |
| Tetrapenta | 11,3 | 4,6 |
| Gesamtausbeute | 924,0 | 95,9 |

| | | |
|---|---|---|
| * siehe Fußnote Beispiel 1 | | |

### Beispiel 7

### Herstellung von Dipentaerythritol, wobei die halbe Penta-Menge vorgelegt und die andere Hälfte während der Reaktion zugegeben wurde.

In einem 6 l-Reaktionsgefäß wurden 272,8 g (2 mol) wäßrige 22 % Formaldehyd-Lösung, 136 g (1 mol) Pentaerythritol, 1600 g (88 mol) Wasser, 96 g (2,4 mol) Natriumhydroxid auf 40 °C temperiert. Zu dieser Lösung wurden innerhalb von 2 h bei einer konstanten Temperatur von 40 °C 116 g (2 mol) Acrolein (96 %) und 1091,2 g (8 mol) 22 % Formaldehyd-Lösung kontinuierlich zudosiert. Gleichzeitig wurden innerhalb 1 h 136 g (1 mol) Pentaerythritol in 1600 g (88 mol) Wasser kontinuierlich zugegeben. Nach der Zugabe wurde das Reaktionsgemisch noch 30 min. bei der Temperatur gerührt und anschließend mit Ameisensäure auf pH = 6 eingestellt. Der Formaldehyd wurde durch Wasserdampfdestillation entfernt und die Zusammensetzung der Reaktionslösung mittels Gaschromatographie analysiert. Es wurde ein Eindampfrückstand von 690 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 2,8 | 1,0 |
| Penta | 367,8 | - |
| Penta (neu) * | 95,8 | 35,2 |
| Dipenta | 89,2 | 35,1 |
| Pe₂fo | 7,1 | 2,5 |
| Tripenta | 23,6 | 9,5 |
| Tetrapenta | 7,1 | 2,9 |
| Gesamtausbeute | 497,6 | 86,2 |

| | | |
|---|---|---|
| * siehe Fußnote Beispiel 1 | | |

### Beispiel 8

### Herstellung von Pentaerythritol-TMP-Ether

In einem 6 l-Reaktionsgefäß wurden 272,8 g (2 mol) 22 % Formaldehyd-Lösung, 268 g (2 mol) TMP, 3200 g (177 mol) Wasser, 96 g (2,4 mol) Natriumhydroxid auf 40 °C temperiert. Zu dieser Lösung wurden innerhalb von 2 h bei einer konstanten Temperatur von 40 °C 116 g (2 mol) Acrolein (96 %) und 1091,2 g (8 mol) 22 % Formaldehyd-Lösung, die vorher über einen Mischer liefen, kontinuierlich zudosiert. Nach der Zugabe wurde das Reaktionsgemisch noch 30 min. bei der Temperatur gerührt und anschließend mit Ameisensäure auf pH = 6 eingestellt. Der Formaldehyd wurde durch Wasserdampfdestillation oder Druckdestillation entfernt und die Zusammensetzung der Reaktionslösung mittels Gaschromatographie analysiert. Es wurde ein Eindampfrückstand von 700 g erhalten. Die Zusammensetzung und Ausbeuten folgen aus der Tabelle:

| Polyol | Zusammensetzung des Rückstandes (g) | Ausbeute bezogen auf Acrolein (%) |
|---|---|---|
| Pefo | 2,7 | 0,9 |
| Penta | 131,0 | 48,0 |
| TMP | 195,3 | - |
| Dipenta | 19,8 | 7,8 |
| TMP-Penta-Ether | 101,4 | 20,1 |
| TMP/Penta-Formale | 9,0 | 2,4 |
| Tripenta | 2,7 | 1,1 |
| TMP-Penta₂-Ether | 18,9 | 5,1 |
| Gesamtausbeute | 480,8 | 85,4 |

## Patentansprüche

1. Verfahren zur Herstellung von Polyhydroxyethern der allgemeinen Formel (I)
(HO)_{(w-m)}W(OV)ₘ (I),
worin W für einen aliphatische und/oder cycloaliphatische Strukturmerkmale aufweisenden organischen Rest eines primäre und/oder sekundäre Hydroxylgruppen enthaltenden organischen mehrwertigen Alkohols der allgemeinen Formel W(OH)_{w} (II), W für eine ganze Zahl von 2 bis 1000 und m für eine ganze Zahl von 1 bis w stehen und OV ausgewählt ist aus hydroxylgruppenhaltigen Strukturelementen der allgemeinen Formel (III) 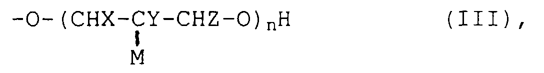 worin bedeuten
X: H, C₁- bis C₆-Alkyl, Aryl, Alkoxymethyl, Aryloxymethyl;
Y: H, C₁- bis C₆-Alkyl, Aryl, Alkoxymethyl, Aryloxymethyl, Carbalkoxymethyl, Carbamidomethyl oder das Strukturmerkmal M;
Z: H, C₁- bis C₆-Alkyl, Aryl oder X-Z zusammen eine C₂- oder C₃-Alkylengruppe;
M: -CH₂OH oder 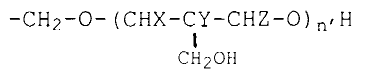
n,n': eine ganze Zahl von 1 bis 5, und
wobei Alkyl und Aryl in X, Y und Z auch substituiert und die m Strukturelemente OV im Polyhydroxyether (I) gleich oder verschieden sein können,
umfassend Umsetzen eines mehrwertigen Alkohols der allgemeinen Formel W(OH)_{w} (II) mit einer α,β-ungesättigten Carbonylverbindung der allgemeinen Formel
XCH=CY-CZ=O (IV),
worin X, Y, und Z mit der Ausnahme, daß Y nicht für das Strukturmerkmal M steht, die vorgenannte Bedeutung haben,
und Formaldehyd in Gegenwart eines Alkali- oder Erdalkalihydroxids in wäßriger Phase bei einem pH-Wert oberhalb 7 und einer Temperatur im Bereich von 0 bis 100 °C, wobei das Molverhältnis von II zu IV 0,05 bis 20, das Molverhältnis von Formaldehyd zu IV 2 bis 15 und das Molverhältnis von Metallhydroxidäquivalent zu IV 1 bis 3 beträgt, Ansäuern des Reaktionsgemischs, und Isolieren der Reaktionsprodukte,
dadurch gekennzeichnet,
daß man in eine wäßrige Lösung, enthaltend mindestens 10 Mol-% des mehrwertigen Alkohols (II), mindestens 10 bis 70 Mol-% des Formaldehyds und mindestens 10 Mol-% des Alkali- oder Erdalkalihydroxids, die α,β-ungesättige Carbonylverbindung (IV) und den restlichen Formaldehyd, den restlichen mehrwertigen Alkohol (II) und restliches Alkali- oder Erdalkalihydroxid kontinuierlich oder periodisch derart einträgt, daß im Reaktionsgemisch Formaldehyd stets im Überschuß gegenüber dem α,β-ungesättigten Aldehyd oder Keton (IV) vorliegt.

2. Verfahren nach Anspruch 1,
dadurch gekennzeichnet,
daß man einen mehrwertigen Alkohol (II) aus der Reihe α,ω-(C₂- bis C₆-)Alkylenglykole, Neopentylglykol, Trimethylolethan (TME), Trimethylolpropan (TMP), Pentaerythritol, Dipentaerythritol, Di-TME und Di-TMP einsetzt.

3. Verfahren nach Anspruch 1 oder 2,
dadurch gekennzeichnet,
daß man eine α,β-ungesättigte Carbonylverbindung (IV) aus der Reihe Acrolein, α-(C₁- bis C₄)-Alkylacrolein, α-Arylacrolein, α-Alkoxymethylacrolein und α-Aryloxymethylacrolein einsetzt.

4. Verfahren nach einem der Ansprüche 1 bis 3,
dadurch gekennzeichnet,
daß man 50 bis 100 Mol-% des mehrwertigen Alkohols (II) in der wäßrigen Lösung oder Suspension vorlegt.

5. Verfahren nach einem der Ansprüche 1 bis 4,
dadurch gekennzeichnet,
daß man 50 bis 100 Mol-% des Alkali- oder Erdalkalihydroxids in der wäßrigen Lösung oder Suspension vorlegt.

6. Verfahren nach einem der Ansprüche 1 bis 5,
dadurch gekennzeichnet,
daß man 20 bis 50 Mol-% des Formaldehyds in der wäßrigen Lösung oder Suspension vorlegt.

7. Verfahren nach einem der Ansprüche 1 bis 6,
dadurch gekennzeichnet,
daß man einen mehrwertigen Alkohol (II) mit 2 bis 6 primären Hydroxylgruppen und einen α,β-ungesättigten Aldehyd (IV) auswählt und im Molverhältnis im Bereich von 5 bis 0,2 umsetzt.

8. Verfahren nach einem der Ansprüche 1 bis 7,
dadurch gekennzeichnet,
daß man 100 Mol-% eines mehrwertigen Alkohols (II) mit 2 bis 6 Hydroxylgruppen, 20 bis 30 Mol-% des Formaldehyds und 100 Mol-% des Alkali- oder Erdalkalihydroxids in einer wäßrigen Lösung vorlegt, einen α,β-ungesättigten Aldehyd (IV) und den restlichen Formaldehyd parallel oder vorgemischt kontinuierlich innerhalb von 0,5 bis 5 h zugibt und die Umsetzung bei 30 bis 60 °C durchführt.

## Claims

1. Process for preparing polyhydroxyethers with the general formula (I)
(HO)_{(w-m)}W(OV)ₘ (I),
wherein W denotes an organic radical exhibiting aliphatic and/or cycloaliphatic structural features of an organic polyhydric alcohol of the general formula W(OH)_{w} (II) containing primary and/or secondary hydroxyl groups, W denotes an integer from 2 to 1000 and m denotes an integer from 1 to w and OV is selected from structural elements containing hydroxyl groups of the general formula (III) 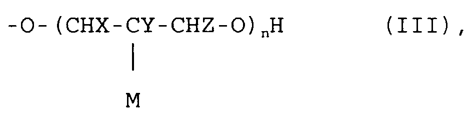 having the following meanings:
X: H, C₁ to C₆ alkyl, aryl, alkoxymethyl, aryloxymethyl;
Y: H, C₁ to C₆ alkyl, aryl, alkoxymethyl, aryloxymethyl, carbalkoxymethyl, carbamidomethyl or the structural feature M;
Z: H, C₁ to C₆ alkyl, aryl or X-Z together are a C₂ or C₃ alkylene group;
M: -CH₂OH or 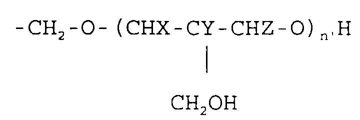
n,n': an integer from 1 to 5, and
wherein alkyl and aryl in X, Y and Z may also be substituted and the m structural elements OV in the polyhydroxyether (I) may be the same or different, comprising reacting a polyhydric alcohol with the general formula W(OH)_{w} (II) with an α,β-unsaturated carbonyl compound with the general formula
XCH=CY-CZ=O (IV),
wherein X, Y and Z have the above meaning, with the exception that Y does not denote the structural feature M, and formaldehyde, in the presence of an alkali or alkaline earth hydroxide in the aqueous phase at a pH greater than 7 and a temperature in the range of 0 to 100°C, the molar ratio of II to IV being 0.05 to 20, the molar ratio of formaldehyde to IV being 2 to 15 and the molar ratio of metal hydroxide equivalent to IV being 1 to 3, acidifying the reaction mixture and isolating the reaction products,
characterised in that,
into an aqueous solution containing at least 10 mole % of the polyhydric alcohol (II), at least 10 to 70 mole % of the formaldehyde and at least 10 mole % of the alkali or alkaline earth hydroxide, the α,β-unsaturated carbonyl compound (IV) and the remaining formaldehyde, the remaining polyhydric alcohol (II) and remaining alkali or alkaline earth hydroxide are introduced continuously or periodically in such a way that formaldehyde is always present in the reaction mixture in excess compared with the α,β-unsaturated aldehyde or ketone (IV).

2. Process according to claim 1,
characterised in that
a polyhydric alcohol (II) from the series α,ω-(C₂ to C₆) alkylene glycols, neopentyl glycol, trimethylolethane (TME), trimethylolpropane (TMP), pentaerythritol, dipentaerythritol, di-TME and di-TMP is used.

3. Process according to claim 1 or 2,
characterised in that
an α,β-unsaturated carbonyl compound (IV) from the series acrolein, α-(C₁ to C₄) alkylacrolein, α-arylacrolein, α-alkoxymethylacrolein and α-aryloxymethylacrolein is used.

4. Process according to one of claims 1 to 3,
characterised in that
50 to 100 mole % of the polyhydric alcohol (II) is initially placed in the aqueous solution or suspension.

5. Process according to one of claims 1 to 4,
characterised in that
50 to 100 mole % of the alkali or alkaline earth hydroxide is initially placed in the aqueous solution or suspension.

6. Process according to one of claims 1 to 5,
characterised in that
20 to 50 mole % of the formaldehyde is initially placed in the aqueous solution or suspension.

7. Process according to one of claims 1 to 6,
characterised in that
a polyhydric alcohol (II) with 2 to 6 primary hydroxyl groups and an α,β-unsaturated aldehyde (IV) are selected and reacted in a molar ratio in the range of 5 to 0.2.

8. Process according to one of claims 1 to 7,
characterised in that
100 mole % of a polyhydric alcohol (II) with 2 to 6 hydroxyl groups, 20 to 30 mole % of the formaldehyde and 100 mole % of the alkali or alkaline earth hydroxide are initially placed in an aqueous solution, an α,β-unsaturated aldehyde (IV) and the remaining formaldehyde are added continuously, in parallel or pre-mixed, within 0.5 to 5 h and the reaction is carried out at 30 to 60°C.

## Revendications

1. Procédé de production de polyhydroxyéthers de formule générale (I)
(HO)_{(w-m)}W(OV)ₘ (I),
dans laquelle W représente un radical organique, présentant des caractères structuraux aliphatiques et/ou cycloaliphatiques, d'un alcool polyvalent organique contenant des groupes hydroxyle primaires et/ou secondaires, de formule générale W(OH)_{w} (II), w représente un nombre entier compris entre 2 et 1000 et m représente un nombre entier compris entre 1 et w, et OV est choisi parmi les éléments structuraux contenant des groupes hydroxyle de formule générale (III) 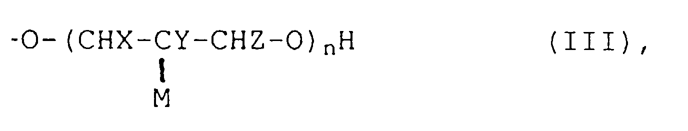 dans laquelle
X représente H, un alkyle en C₁ à C₆, un aryle, un alcoxyméthyle, un aryloxyméthyle ;
Y représente H, un alkyle en C₁ à C₆, un aryle, un alcoxy méthyle, un aryloxyméthyle, un carbalcoxyméthyle, un carbamidométhyle ou l'élément structural M ;
Z représente H, un alkyle en C₁ à C₆, un aryle ou X-Z ensemble représentent un groupe alkylène en C₂ ou C₃ ;
M représente -CH₂OH ou 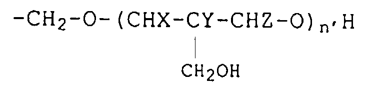
n,n' : représentent un nombre entier compris entre 1 et 5, et où
alkyle et aryle dans X, Y et Z peuvent également être substitués et les m éléments structuraux OV dans le polyhydroxyéther (I) peuvent être identiques ou différents,
comprenant la réaction d'un alcool polyvalent de formule générale W(OH)_{w} (II) avec un composé carbonyle α,β-insaturé de formule générale
XCH=CY-CZ=O (IV),
dans laquelle X, Y et Z, sous réserve que Y ne représente pas le caractère structural M, ont la signification indiquée plus haut,
et le formaldéhyde en présence d'un hydroxyde de métal alcalin ou de métal alcalino-terreux en phase aqueuse a un pH supérieur à 7 et a une température comprise entre 0 et 100°C, le rapport molaire de II à IV étant compris entre 0,05 et 20, le rapport molaire du formaldéhyde à IV étant compris entre 2 et 15 et le rapport molaire de l'équivalent hydroxyde métallique à IV étant compris entre 1 et 3, avec ensuite l'acidification du mélange réactionnel et l'isolement des produits de la réaction,
caractérisé en ce que
dans une solution aqueuse contenant au moins 10 % molaire de l'alcool polyvalent (II), au moins de 10 à 70 % molaire du formaldéhyde et au moins 10 % molaire de l'hydroxyde de métal alcalin ou alcalino-terreux, on introduit de façon continue ou périodique le composé carbonyle α,β-insaturé (IV) et le reste de formaldéhyde, le reste de l'alcool polyvalent (II) et le reste de l'hydroxyde de métal alcalin ou alcalino-terreux, en ce que dans le mélange réactionnel, le formaldéhyde est toujours en excès par rapport à l'aldéhyde ou à la cétone α,β-insaturé(e) (IV).

2. Procédé selon la revendication 1,
caractérisé en ce qu'
on utilise un alcool polyvalent (II) de la série α,ω-(alkylène en C₂ à C₆) glycols, néopentylglycol, triméthyloléthane (TME), triméthylolpropane (TMP), pentaérythritol, dipentaérythritol, di-TME et di-TMP.

3. Procédé selon la revendication 1 ou 2,
caractérisé en ce qu'
on utilise un composé carbonyle α,β-insaturé (IV) de la série acroléine, α-(alkyle en C₁ à C₄)acroléine, α-arylacroléine, α-alcoxyméthylacroléine et α-aryloxyméthylacroléine.

4. Procédé selon l'une des revendications 1 à 3,
caractérisé en ce qu'
on dispose dans la solution ou suspension aqueuse de 50 à 100 % molaire de l'alcool polyvalent (II).

5. Procédé selon l'une des revendications 1 à 4,
caractérisé en ce qu'
on dispose de 50 à 100 % molaire de l'hydroxyde de métal alcalin ou alcalino-terreux dans la solution ou suspension aqueuse.

6. Procédé selon l'une des revendications 1 à 5,
caractérisé en ce qu'
on dispose de 20 à 50 % molaire de formaldéhyde dans la solution ou suspension aqueuse.

7. Procédé selon l'une des revendications 1 à 6,
caractérisé en ce qu'
on choisit un alcool polyvalent (II) avec de 2 à 6 groupes hydroxyle primaires et un aldéhyde α,β-insaturé (IV) et en ce qu'on les fait réagir dans un rapport molaire compris entre 5 et 0,2.

8. Procédé selon l'une des revendications 1 à 7,
caractérisé en ce qu'
on dispose 100 % molaire d'un alcool polyvalent (II) avec de 2 à 6 groupes hydroxyle, de 20 à 30 % molaire de formaldéhyde et 100 % molaire de l'hydroxyde de métal alcalin ou alcalino-terreux dans une solution aqueuse, en ce qu'on ajoute un aldéhyde α,β-insaturé et en ce qu'on ajoute parallèlement ou sous forme prémélangée le reste du formaldéhyde de façon continue en 0,5 à 5 h, et en ce qu'on conduit la réaction entre 30 et 60° C.
